## Europäisches Patentamt

## European Patent Office

## Office européen des brevets

(11) Publication number: **0 081 099**
**A2**

(12)

# EUROPEAN PATENT APPLICATION

(21) Application number: **82110494.0**

(51) Int. Cl.³: **C 07 H 21/00**

(22) Date of filing: **13.11.82**

(30) Priority: **04.12.81 US 327523**

(43) Date of publication of application:
**15.06.83 Bulletin 83/24**

(84) Designated Contracting States:
**DE FR GB IT**

(71) Applicant: **Sloan-Kettering Institute For Cancer Research**
**1275 York Avenue**
**New York New York 10021(US)**

(72) Inventor: **Krug, Robert M.**
**361 Whitman Street**
**Haworth New Jersey 07641(US)**

(72) Inventor: **Plotch, Stephen J.**
**95 West 95th Street**
**New York New York 10025(US)**

(74) Representative: **Patentanwälte Schulze Horn und Hoffmeister**
**Goldstrasse 36**
**D-4400 Münster(DE)**

(54) **Capped oligonucleotide anti-viral agents.**

(57) Capped oligonucleotide anti-viral agent comprising: a cap having the formula m⁷G (5')-ppp (5') Nm, where N=A or G and m=2' O-methyl ribose, a linkage group X, attached to said cap, where X=A, G, C or U, a polymeric chain attached to said linkage group comprised of nucleotides that inhibit influenza virus RNA transcription when in polymer form, said polymeric chain being modified to prevent it from acting as a primer to stimulate transcription.

EP 0 081 099 A2

The invention described herein was made in the course of work under a grant or award from the Department of Health, Education and Welfare.

BACKGROUND

The invention relates to new capped oligonucleotide compounds effective at low concentrations ($10^{-9}$M) in inhibiting the influenza virion transcriptase. These compounds have the potential of being useful in the treatment of influenza virus infections.

Uncapped ribopolymer compounds having specific inhibitory activity for the influenza virion are known from Krug et. al., Proc. Natl. Acad. Sci. U. S. A. 77, 5874 (1980); Smith et. al., Virology 103, 245 (1980) and Plotch et. al., Cell 23, 847 (1981). However, these compounds require concentrations of $10^{-7}$M to produce 90 % inhibition of the transcriptase in vitro.

It is believed important to obtain compounds which are effective inhibitors (90 % inhibition) at a 100-fold lower concentration than that required by the previously described uncapped ribopolymers inhibitors both to reduce the possibility of toxic side effects and to increase the likelihood of achieving effective intracellular concentrations should there be difficulty in these compounds being taken up by cells.

## SUMMARY

The objects of the invention are accomplished by providing certain capped oligonucleotides. The oligonucleotide is made up of bases (nucleotides) which when present in uncapped oligonucleotides inhibit the influenza virion transcriptase (see Description). The 5' terminal cap is provided to increase the affinity of the oligonucleotide for the virion transcriptase and thereby increase inhibitory activity. To prevent these capped oligonucleotides from priming (i. e., stimulating) rather than inhibiting the transcriptase, it is intended: (1) to make the oligonucleotide less than 10 nucleotides in length and containing a 3'terminal deoxymononucleotide or 3'-O-methylated ribomononucleotide so that it cannot be utilized as a primer (see Description); or (2) with oligonucleotides longer than 10 nucleotides, to modify the oligonucleotide (in the sugar and/or base moieties and/or in the nucleotide bond) so that either they cannot be cleaved at the 10 - 13th nucleotide bond by the virion endonuclease or, if they can be cleaved, cannot act as primers. These modifications will also be introduced into the capped oligonucleotides shorter than 10 nucleotides in length, if it is necessary to eliminate any residual priming activity in these oligonucleotides.

## FIGURES

Figure 1 schematically illustrates the blockage of enzyme cleavage or the blockage of utilization as primer by steric hindrance effects, the cleavage site being indicated by the arrow;

Figure 2 schematically illustrates the blockage of enzyme cleavage or blockage of utilization as a primer by changing the chemical nature of the sugar moiety, the cleavage site being indicated by the arrow; and

Figure 3 schematically illustrates the blockage of enzyme cleavage or blockage of utilization as primer by modifying the position or chemistry of the cleavage site shown by the arrow.

Glossary

$m^7G$: 7-methyl guanosine (ribonucleoside)
A: adenosine (ribonucleoside) ·
U: uridine (ribonucleoside)
C: cytidine (ribonucleoside)
G: guanosine (ribonucleoside)
$s^4U$: 4-thio uridine (ribonucleoside)
m: 2'-O-methyl ribose
d: deoxyribose (i. e., 2'-H ribose)

DESCRIPTION

It has been shown that eukaryotic messenger RNAs (and any RNA containing a 5' terminal methylated cap structure, $m^7G$ (5') ppp (5') Nm, where N usually is A or G) act as primers for the synthesis of influenza viral messenger RNA and donate their cap structure and a short stretch of nucleotides to the viral messenger RNA molecules. Bouloy et. al., Proc. Natl. Acad. Sci. U. S. A. 75, 4886-4890 (1978); Bouloy et al., J. Virol. 32, 895 - 904 (1979); Plotch et. al., Proc. Natl. Acad. Sci. U. S. A. 76, (1618 - 1622 (1979); Robertson et. al., Nucleic Acids Res., 8, 925 - 942 (1980); Krug et. al., Proc. Natl. Acad. Sci. U. S. A., 77, 5874 - 5878 (1980);

Plotch et. al., Cell 23, 847 - 858 (1981). This priming reaction does not involve hydrogen-bonding between the primer and the template virion RNA. Instead, the stimulation of the initiation of transcription most likely results from a specific interaction between the primer RNA and one or more proteins associated with the viral transcriptase complex. This specific interaction apparently involves the 5' methylated cap structure, since priming activity is dependent on the presence of this cap structure. The first step in the reaction is the cleavage of the primer RNA by an endonuclease which is found in purified viral cores. This unique enzyme requires the presence of a methylated cap structure in the RNA substrate and cleaves preferentially at a purine residue 10 to 13 nucleotides from the cap, generating fragments with a 3' hydroxyl group. These fragments are the actual primers that initiate transcription. Cleavage of the RNA primer and initiation of transcription are not necessarily coupled, because a 5' fragment isolated from one reaction could be used as a primer when added to a second reaction. The nuclease-generated capped fragments bind efficiently to the viral transcriptase complex (Ulmanen et. al., Proc. Natl. Acad. Sci. U. S. A. 78 (1981), in press). Capped fragments as short as seven nucleotides in length plus the cap (i. e., shorter than the endonuclease-generated fragments) also bind efficiently to the transcriptase complex, but are poorly, if at all, utilized as primers. Very short capped fragments (one or two nucleotides long), however, bind poorly to the transcriptase complex.

It has also been shown that influenza viral RNA transcription is inhibited by certain uncapped ribopolymers with reduced secondary structure; poly $S^4U$ and poly AG being the most effective of those tested Krug et. al., Proc. Natl. Acad. Sci. U. S. A. (1980) supra; Plotch et. al., Cell (1981) supra. Results indicate that the nucleotides which, when found in an environment free of intra- and intermolecular hydrogen-bonding, contribute to the inhibitory activity of a polyribonucleotide are U, $S^4U$ and G, but not A and C. The former group of nucleotides contain an oxygen or sulfur (rather than amino) group at equivalent sites on the purine (6-position) or pyrimidine (4-position) ring.

A major object of the invention is to synthesize compounds which have a considerably higher specific inhibitory activity than the uncapped ribopolymers. The latter require a concentration of about $10^{-7}$ M to produce a 90 % inhibition of the influenza virion transcriptase _in vitro_. The inventive compounds should produce the same level of inhibition with at least a 100-fold lower concentration (in the range of $10^{-9}$ M). This is accomplished by synthesizing oligonucleotides with the following characteristics: (1) contain a 5' methylated cap structure to increase the affinity of the oligonucleotide for the virion endonuclease and transcriptase; and (2) contain those bases which habe been shown to contribute to the inhibitory activity of uncapped ribopolymers. In addition, modifications must be performed to prevent these capped oligonucleotides from being primers rather than inhibitors. Without such modifications, the inventive compounds would be expected, from the prior art, to act as primers - and in fact this has been found to be the case. One alternative will be to make the capped oligonucleotide less than 10 nucleotides in length (probably about 6 to 8 nucleotides in length) so that it will bind effectively to the transcriptase complex, but will not be used as a primer. To ensure that these oligonucleotides would not act as primer, they will also be extended to contain a 3' terminal deoxymononucleotide (e. g., pdCp), or a 3' terminal 3'-O-methylated ribomononucleotide pC(3'O-methyl).

Another alternative will be to synthesize capped oligonucleotides at least 14 nucleotides in length which will be modified in the sugar and/or base moities and/or in the nucleotide bond so that either they cannot be cleaved by the virion endonuclease or, if they can be cleaved, cannot act as primers for viral RNA transcription. It is noted that the modifications mentioned above are important aspects in the synthesis of the inventive compounds. These modifications will also be introduced into the capped oligonucleotides less than 10 nucleotides in length, if it is necessary to eliminate any residual priming activity.

The inventive compounds are oligonucleotides formed by attaching polymeric bases to a capped fragment having the formula $m^7G$ (5') ppp (5') NmpX, where N = A or G and X = A, G, C or U and m = 2'O-methyl ribose. The linkage group pX allows attachment of the polymeric bases. In order to synthesize sufficient quantities of the desired capped oligonucleotides, it will first be necessary to synthesize 50 - 200 mg of a representative capped fragment, i. e., $m^7G$ (5') ppp (5') AmpC. Methods for the synthesis of this compound have been published (Yamaguchi et. al., Nucleic Acids Res. Sp. publ. 2, 5151 (1976)). This is the smallest molecule that can serve as acceptor for RNA ligase or as primer for polynucleotide phosphorylase, which are the two enzymes which will be used to extend this capped fragment. Using the small amount of $m^7$GpppGmpC which can be derived from reovirus messenger RNAs by digestion with ribonuclease T2 and alkaline phosphatase, optimum conditions have been determined for these two enzymes. Examples of capped oligonucleotide inhibitors are given below.

Example 1

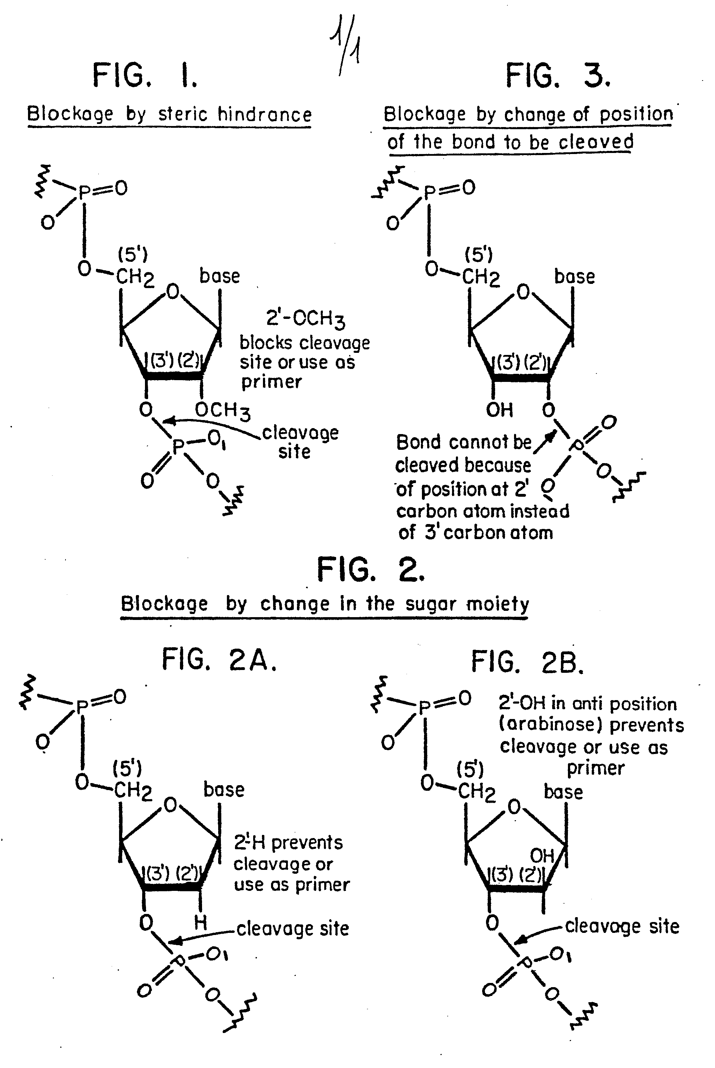

Cleavage of the capped oligonucleotide can be prevented by blocking the cleavage site (see Figure 1). One preferred method is to employ modified sugars with a 2'-O-methyl group e. g., $m^7$GpppAmpC (2'-O-Me $S^4$U)$_n$ where n is greater than 11. This is accomplished either by incubating $m^7$GpppAmpC with 2'-O-methylated $S^4$UDP (available commercially from P-L Biochemicals) in the presence of M. Luteus polynucleotide phosphorylase under primer-dependent conditions or by using RNA ligase to link $m^7$GpppAmpC to 5' phosphorylated ribooligonucleotides of 2'-O-methylated $S^4$U (synthesized with E. coli. or M. luteus polynucleotide phosphorylase under primer-independent conditions followed by dephosphorylation with alkaline phosphatase and 5' phosphorylation with polynucleotide kinase) (Both et. al., J. Mol. Biol. 104, 637 (1976); England and Uhlenbeck, Biochemistry 17, 2069 (1978); Higgins et. al., Nucleic Acids Res., 6, 1013 (1979)). If cleavage is not blocked, the compound may still act as an inhibitor by virtue of the inability of the modified oligonucleotide to act as a primer.

Example 2

A) Cleavage of the capped oligonucleotide can be prevented by changing the chemical nature of the sugar moiety (see Figure 2 A). One preferred method is to employ modified sugars with 2'-H groups (deoxyoligonucleotides). An example of this type of compound is $m^7$GpppAmpC (dS$^4$U)n where n > 11. $m^7$GpppGmpC is first linked to á deoxynucleoside 3', 5' diphosphate, e. g., pdAp, using RNA ligase. This enzyme uses deoxy as well as ribo donors (Hinton et. al., Biochemistry 17, 5091 (1978); Hinton and Gumport, Nucleic Acids Res. 7, 453 (1979)). Following phosphatase treatment to generate a 3'-OH group, the molecule is extended by incubating it in the presence of terminal transferase and deoxynucleoside(s) triphosphates of choice (Bollum, The Enzymes 10, 145 (1974); Roychoudhury et. al., Nucleic Acids Res. 3, 863 (1976)). As an alternative approach (which allows better control of the length of the deoxynucleotides added), we link $m^7$GpppAmpC to 5' phosphorylated deoxyoligonucleotides (available commercially from P-L Biochemicals) of the desired length (n > 11) using RNA ligase. Here too, if cleavage is not blocked, the compound may still act as an inhibitor by virtue of the inability of the modified oligonucleotide to act as a primer.

B) Another preferred method is to employ capped oligonucleotides containing arabinose rather than ribose as the sugar moiety (see Figure 2 B). For the synthesis of these compounds, we will link $m^7$GpppAmpC to 5' phosphorylated arabino-oligonucleotides of the desired length (n > 11) using RNA ligase. An example of these compounds is $m^7$GpppAmpC $(Ara-X)_n$ where X = A, C or U.

Example 3

Cleavage of capped oligonucleotides can be prevented by modifying the position or chemistry of the internucleotide bond at the cleavage site. Because the enzyme cleavage is so specific, this prevents the enzyme from recognizing the site.

Thus, as the influenza virion endonuclease generates capped fragments with 3'-hydroxyl groups, changing the internucleotide linkage from 3', 5' to 2', 5' nucleotide bonds would probably prevent cleavage (see Figure 3). One approach to the synthesis of this type of capped oligonucleotide is to prepare 2', 5' oligonucleotides, like (ppp) ApApA or (ppp) ApApG, using the 2'-5' oligoadenylate synthetase (Justesen et. al.,Proc. Natl.

Acad. Sci. U. S. A. 77, 4618 (1980)), and the chemically link these fragments together via 2', 5' bonds in order to generate oligonucleotides longer than 11 nucleotides in length. This chemical reaction is rather straightforward. These oligonucleotides are then linked (via a 3', 5' bond) to $m^7$GpppCmpC using RNA ligase. An example of this type of compound is $m^7$GpppAmpC (2', 5'A)$_n$ where n > 11. If cleavage is not blocked, the compound may still act as an inhibitor by virtue of the inability of the modified oligonucleotide to act as a primer.

Example 4

Another way to change the chemistry of the site and thus prepare oligonucleotide inhibitors is by modifying the component bases. This takes advantage of the specificity exhibited by the virion endonuclease for cleavage at particular nucleotides. Whith capped RNAs containing a relatively even distribution of purines and pyrimidines at their 5' end, cleavage occurs almost exclusively at purines. The endonuclease, however, is able to cleave at U residues, as the ribopolymer $m^7$GpppGmpC(U)$_n$ exhibits some priming activity, but apparently this enzyme cannot cleave at C residues, as $m^7$GpppGmpC(C)$_n$ exhibits minimal or no priming activity. Thus base modifications may block cleavage by the virion endonuclease. A representative structure of such a base - modified capped oligonucleotide is

$$m^7\text{GpppAmpC(B)}_n$$

where n > 11, and B represents modified bases produced by reacting the uncapped polyribonucleotide under the following conditions (after which the modified-poly-nucleotide would be linked to the cap structure $m^7$GpppAmpC with RNA ligase):

a) reaction of poly $S^4$U with acrylonitrile to form the S-cyanoethyl derivative (Chambers, Biochemistry 4, 219 (1965).

b) reaction of poly $S^4U$ with ethyleneimine or N-ethyl-maleimide to form the respective S-substituted derivatives (Carbon and David, Biochemistry 7, 3851 (1968); Reid, Biochem. Biophys. Res. Commun. 33, 627 (1968)).

c) Kethoxylation and glyoxylation of the G residues in poly AG (Litt and Hancock, Biochemistry 6, 1848 (1967); Litt, Biochemistry 8, 3249 (1969)).

d) methylation of the N-7 position of G and the 1-position of A in poly AG with dimethyl sulfate (Pillinger et. al., Biochem. J. 114, 429 (1969); Hay, Biochem. J. 114, 28P (1969)).

Combinations of Examples 1 - 4

To enhance the effectiveness of base modifications to produce useful inhibitors, capped oligonucleotide inhibitors containing combinations of modified (and unmodified) bases and modified sugar moieties and/or internucleotide linkages can be used. Three examples of these classes of compound are $m^7GpppAmpC$ (2'-O-Me $S^4$-cyanoethyl U)$_n$, $m^7GpppAmpC$ (2'HN1-methyl A, N7-methyl G)$_n$, and $m^7GpppAmpC$ (2'-5N1-methyl A)$_n$ where n > 11.

Example 5

Because the influenza virus transcriptase can bind capped oligonucleotides 7 to 13 or more nucleotides in length but only efficiently utilizes as primer capped oligonucleotides 10 to 13 nucleotides in length, capped oligonucleotides 7 to 9 nucleotides in length should act as specific inhibitors. The addition of a 3' terminal deoxymononucleotide or a 3' terminal 3'-O-

methylated ribooligonucleotide to such oligonucleotides would ensure that they act as an inhibitor rather
than a primer. Examples of this type of compounds is
$m^7$GpppAmpC $(S^4U)_5$pdCp and $m^7$GpppAmpC $(S^4U)_5$pC(3'O-methyl).
The synthesis of these compounds are accomplished by
linking $p(S^4U)_5$ (synthesized using $S^4$UDP, polynucleotide,
phosphorylase, alkaline phosphatase, and polynucleotide
kinase as described previously) to $m^7$GpppAmpC using RNA
ligase followed by linking pdCp to $m^7$GpppAmpC $(S^4U)_5$
using RNA ligase or reacting (3'O-methyl) CDP with
$m^7$GpppAmpC $(S^4U)_5$ using polynucleotide phosphorylase.


Combinations of Example 5 with Examples 1-3


Effective inhibitors of the type containing capped
oligonucleotide inhibitors 7 to 9 nucleotides in length,
which in addition to having a 3' terminal deoxyribo-
mononucleotide or a 3' terminal 3'-O-methylated mononucleotide, can also be prepared containing modified
bases and/or modified sugar moieties and/or internucleotide linkages. Two examples of these kinds of compounds
are

$$m^7\text{GpppAmpC } (2'-5'A)_6\text{pdCp}$$

and

$$m^7\text{GpppAmpC } (2'\text{O-methyl } S^4U)_6\text{pC}(3'\text{O-methyl}).$$


In general, the syntheses reported in the above examples
are monitored in two ways: either using labeled ribo-
or deoxyribonucleotides or using labeled $m^7$GpppGmpC.
The latter will be prepared as we have done previously
with capped RNAs. The $m^7$G of $m^7$GpppGmpC is removed by
beta-elimination followed by recapping with vaccinia
virus capping and methylating enzymes in the presence
of (alpha-$^{32}$P) GTP and unlabeled S-adenosylmethionine
(Plotch et. al., Cell (1981), supra).

DISCUSSION

Although it was believed that the most effective inhibition of _in vitro_ transcription with uncapped ribopolymers required that they be 10 nucleotides or longer in length, the inventive capped oligonucleotides are effective even when as short as 13 nucleotides long. This is because capped RNA fragments (generated from primer RNAs by the virion endonuclease) in this size range are active as primers. In addition, capped oligonucleotides as short as 6 - 8 nucleotides long are also believed to be effective because of their ability to bind to viral cores. Thus, important features of the inventive compounds are the reduction in the concentration and the size of the compounds which are effective with respect to inhibiton of the influenza viral RNA transcriptase.

The inventive compounds are useful in the control of influenza viral infections by their inhibition of transcriptase activity. Also, because of their small size and effective concentration, problems with cell uptake of these compounds are attenuated. To facilitate cell uptake, the capped oligonucleotides can be incorporated into liposomes (Wilson et al., Cell 17, 77 (1979)), which can be made into a pharmaceutically acceptable carrier. In addition, these compounds will not be limited in their usefulness against any one strain of influenza virus, unlike currently available vaccines which operate against a constantly shifting pattern of viral surface antigens, but will be directed against a vital viral replication function common to all present and future viral strains.

WHAT IS CLAIMED IS

1. Capped oligonucleotide comprising:
a cap having the formula $m^7G$ (5') ppp (5') Nm,
where N = A or G and m = 2'O-methyl ribose
a linkage group X, where X = A, G, C or U attached
to said cap a polymeric chain attached to said
linkage group
comprised of nucleotides that inhibit influenza
virus RNA transcription when in polymer form, said
polymeric chain being modified to prevent it, when
attached to the cap from acting as a primer to stimulate

2. Oligonucleotide of claim 1 wherein said polymeric
chain is modified to prevent cleavage at the $10^{th}$
to $13^{th}$ nucleotide by the virion-associated endonuclease.

3. Oligonucleotide of claim 1 wherein said polymeric
chain is modified so that, if cleaved, the cleavage
fragments cannot act as primers.

4. Oligonucleotide of claim 1 having a formula
$m^7G$ (5') ppp (5') NmpX $(B)_n$
where        n > 11
and        B = $S^4U$; $S^4$ cyanoethyl-U;
$S^4$-ethylenimine-U; $S^4$-(N-ethyl-maleimide)-U;
$AG_{1:1}$ (Kethoxylated); $AG_{1:1}$ (glyoxylated);

$AG_{1:1}$ $\begin{matrix} \text{(n-7 methyl G)} \\ \text{(n-1 methyl A)} \end{matrix}$ ; and $AG_{1:1}$.

5. Oligonucleotide of claim 1 wherein said nucleotide
is modified at the sugar moiety.

6. Oligonucleotide of claim 5 wherein said sugar
modification consists of 2'-O-methyl; 2'H; or the sugar is arabinose instead of ribose.

7. Oligonucleotide of claim 1 further comprising an internucleotide linkage of 2' - 5' phosphate in place of 3' - 5' phosphate normally present.

8. Oligonucleotide of claim 1 wherein said polymeric chain attached to said linkage group is comprised of 5 - 7 nucleotides to which is attached at its 3' terminus either a deoxymononucleotide or a 3'-O-methylmononucleotide, said capped oligonucleotide acting as a specific inhibitor for the influenza virion transcriptase while at the same time being unable to act as primer by virtue of its 3' terminal nucleotide and its short length of less than 10 oligoribonucleotides.

9. Oligonucleotide of claim 8 having the formula
   $m^7G$ (5') ppp (5') $NmpX$ $(B)_n M$
   where
   $B = S^4U$; $S^4$-ethylenimine-U; $S^4$-(N-ethylmaleimide)-U: $AG_{1:1}$ (kethoxylated); $AG_{1:1}$ (glyoxylated);

   $AG_{1:1}$ $\begin{array}{l}\text{(n-7 methyl G)}\\ \text{(n-1 methyl A)}\end{array}$ ;

   and $AG_{1:1}$.
   n = 5, 6 or 7

   M = pdAp, pdGp, pdCp, pdUp
       or pA(3'O-methyl), pG(3'O-methyl),
           pC(3'O-methyl), pU(3'O-methyl).

10. Oligonucleotide of claim 8 wherein said nucleotide is modified at the sugar moiety.

11. Oligonucleotide of claim 10 wherein said sugar modification consists of 2'-O-methyl; 2'H; or the sugar is arabinose instead of ribose.

12. Oligonucleotide of claim 8 further comprising an internucleotide linkage of 2'-5' phosphate in place of 3'-5' phosphate normally present.

13. Antiviral agent comprising an effective amount of the oligonucleotide of claim 1 in a pharmaceutically acceptable carrier.

14. Method of inhibiting influenza virion transcriptase comprising applying an effective amount of the oligonucleotide of claim 1 to influenza virus-infected cells.

0081099

1/1

## FIG. 1.

Blockage by steric hindrance

2'-OCH₃ blocks cleavage site or use as primer

cleavage site

## FIG. 3.

Blockage by change of position of the bond to be cleaved

Bond cannot be cleaved because of position at 2' carbon atom instead of 3' carbon atom

## FIG. 2.

Blockage by change in the sugar moiety

### FIG. 2A.

2'-H prevents cleavage or use as primer

cleavage site

### FIG. 2B.

2'-OH in anti position (arabinose) prevents cleavage or use as primer

cleavage site